# EUROPEAN PATENT APPLICATION

(11) **EP 3 943 027 A1**
(43) Date of publication of application: **26.01.2022**
(21) Application number: 21187121.5
(22) Date of filing: 22.07.2021
(51) Int. Cl.: A61B 17/34

(54) **TROCAR SYSTEM WITH FORCE SENSING**

(30) Priority: 22.07.2020 US 202016935808
(71) Applicant: Covidien LP, Mansfield, MA 02048 (US)
(72) Inventor: DESJARDIN, Kevin, M., Prospect, CT 06712 (US); LOBO, Astley C., West Haven, CT 06516 (US)
(74) Representative: Maschio, Antonio

(57) **Abstract**

A trocar system includes a trocar assembly and a force sensor system. The trocar assembly includes a housing and an elongated cannula that extends from the housing. The force sensor system includes a force sensor supported on the trocar assembly, a control unit disposed in electrical communication with the force sensor, a processor, and a memory. The memory has instructions stored thereon, which when executed by the processor, cause the trocar system to sense, with the force sensor, a force applied through the trocar system, determine, with the control unit, if the force exceeds a predetermined threshold, and selectively output a signal through the control unit that indicates that the force exceeds the predetermined threshold.

## Description

### TECHNICAL FIELD

This disclosure relates generally to surgical instruments, and in particular, to trocars for use during a minimally invasive surgical procedure.

### BACKGROUND

Clinicians often use trocars during minimally invasive surgery to gain access to a body cavity within a patient. In order to reach difficult locations or a portion of a surgical field that is farther than the length of a minimally invasive instrument (e.g., a surgical stapler), rather than moving the location of the trocar, a clinician may apply pressure through the instrument to attempt to reach a desired location. This exertion of force may impart undesired pressure through the trocar to a port site location.

### SUMMARY

In order to prevent damage to the port site location (or the patient in general), or to otherwise alert a clinician that they are applying excessive force to the patient, this disclosure details trocar systems including force sensor apparatus that alert a clinician when a force applied through the trocar system exceeds a threshold force.

In accordance with one aspect, this disclosure is directed to a trocar system. The trocar system includes a trocar assembly and a force sensor system. The trocar assembly includes a housing and an elongated cannula extending from the housing. The force sensor system includes a force sensor supported on the trocar assembly, a control unit disposed in electrical communication with the force sensor, a processor, and a memory. The memory has instructions stored thereon, which when executed by the processor, cause the trocar system to sense, with the force sensor, a force applied through the trocar system, determine, with the control unit, if the force exceeds a predetermined threshold, and selectively output a signal through the control unit that indicates that the force exceeds the predetermined threshold.

In aspects, the force sensor may be a piezoelectric sensor.

In aspects, the force sensor may be supported on the housing of the trocar assembly. The housing may include an upper housing portion and a lower housing portion. The force sensor may be sandwiched between the upper and lower housing portions. The force sensor may be supported on an upper surface of the housing.

In aspects, the elongated cannula may support a stopper that is configured to limit insertion depth of the trocar assembly into tissue. The stopper may support the force sensor. The control unit may be supported on the stopper.

In aspects, the control unit may be supported on the housing.

In aspects, the trocar system may include an obturator that is selectively received within trocar assembly.

In aspects, the control unit may be supported on the obturator.

According to yet another aspect, this disclosure is directed to a trocar system including an obturator, a trocar assembly configured to receive the obturator therein, and a force sensor system operatively coupled to one or both of the obturator and the trocar assembly. The force sensor system includes a force sensor ring, a control unit disposed in electrical communication with the force sensor ring, a processor, and a memory. The memory has instructions stored thereon, which when executed by the processor, cause the trocar system to sense, with the force sensor ring, a force applied through the trocar system, determine, with the control unit, if the force exceeds a predetermined threshold, and through the control unit, selectively output a signal that indicates that the force exceeds the predetermined threshold.

In aspects, the force sensor ring may be a piezoelectric sensor.

In aspects, the force sensor ring may be supported on a housing of the trocar assembly. The housing may include an upper housing portion and a lower housing portion. The force sensor ring may be sandwiched between the upper and lower housing portions.

In aspects, the force sensor ring may be supported on an upper surface of the housing and positioned for engagement with the obturator.

In aspects, the trocar assembly may include an elongated cannula that supports a stopper configured to limit insertion depth of the trocar assembly into tissue. The stopper may support the force sensor ring on a bottom surface thereof. The control unit may be supported on the stopper. The control unit may be supported on a housing of one or both of the obturator and the trocar assembly.

The details of one or more aspects of this disclosure are set forth in the accompanying drawings and the description below. Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate embodiments of the disclosure and, together with the detailed description of the aspects given below, serve to explain the principles of the disclosure.
FIG. 1 is a perspective view of one trocar system inserted into tissue in accordance with the principles of this disclosure;
FIG. 2 is a perspective view of a trocar assembly of the trocar system of FIG. 1;
FIG. 3 is an enlarged, perspective view of the indicated area of detail shown in FIG. 2;
FIG. 4 is an enlarged, perspective view, with parts separated, of a proximal portion of the trocar assembly of FIG. 2;
FIG. 5 is an enlarged, cross-sectional view taken along line 5-5 shown in FIG. 2;
FIG. 5A is a flow chart illustrating a method for determining whether force applied through a trocar system exceeds a predetermined threshold;
FIG. 6 is a perspective view of another trocar system in accordance with the principles of this disclosure;
FIG. 7 is an enlarged, perspective view, with parts separated, of a proximal portion of the trocar system of FIG. 6;
FIG. 8 is a perspective view of a portion of a trocar assembly of the trocar system of FIG. 6;
FIG. 9 is a perspective view, with parts separated, of a proximal portion of an obturator assembly of the trocar system of FIG. 6;
FIG. 10 is a perspective view of the proximal portion of the obturator assembly of FIG. 9;
FIG. 11 is an enlarged, cross-sectional view taken along line 11-11 shown in FIG. 6;
FIG. 12 is a perspective view of yet another trocar system inserted into tissue in accordance with the principles of this disclosure;
FIG. 13 is a perspective view of the trocar system of FIG. 12;
FIG. 14 is an enlarged, perspective view, with parts separated, of a sensor system of the trocar system of FIG. 12; and
FIG. 15 is an enlarged, cross-sectional view taken along line 15-15 shown in FIG. 12.

### DETAILED DESCRIPTION

Aspects of this disclosure are described in detail with reference to the drawings, in which like reference numerals designate identical or corresponding elements in each of the several views. Additionally, the term "proximal" or "trailing" refers to the portion of structure that is closer to the clinician and the term "distal" or "leading" refers to the portion of structure that is farther from the clinician. As commonly known, the term "clinician" refers to a doctor (e.g., a surgeon), a nurse, or any other care provider and may include support personnel.

In the following description, well-known functions or constructions are not described in detail to avoid obscuring this disclosure in unnecessary detail.

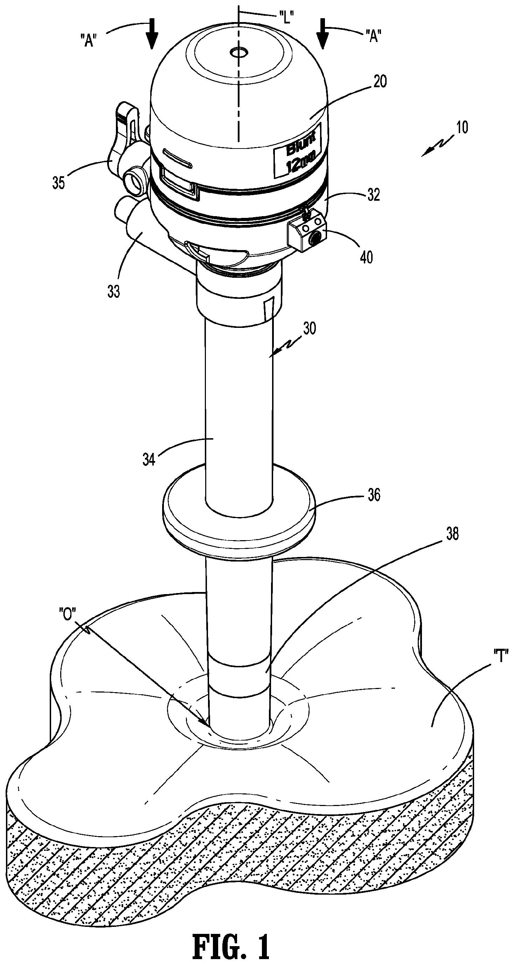

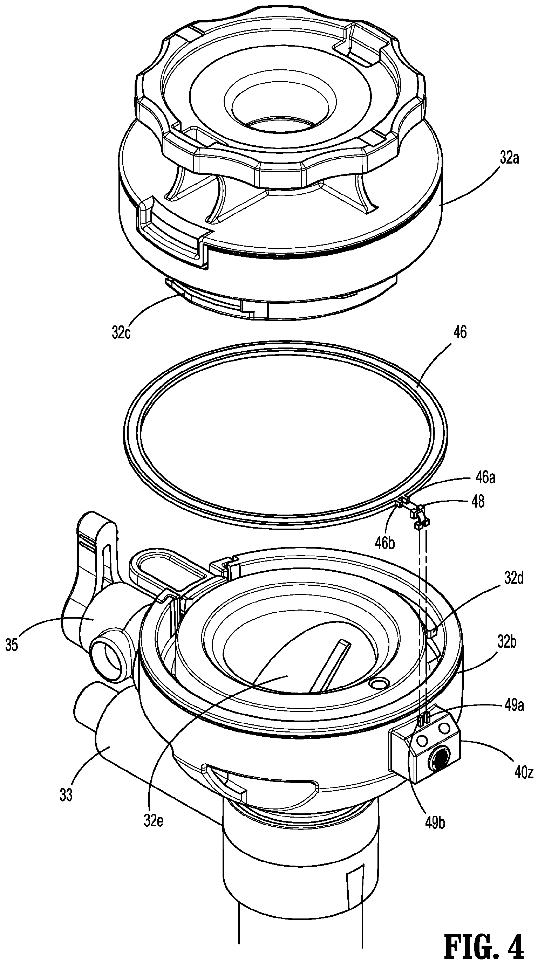

With regard to FIGS. 1-5, a trocar system 10 for selectively accessing a body cavity through tissue "T" is shown. Trocar system 10 defines a longitudinal axis "L" therethrough. Trocar system 10 includes an obturator 20 and a trocar assembly 30 that defines a central passage 31 therethrough for selectively receiving obturator 20 therein. Trocar assembly 30 includes a housing 32 on a proximal end portion thereof and a cannula 34 that extends from housing 32 on a distal end portion thereof. Housing 32 includes an upper housing portion 32a and a lower housing portion 32b that are removably coupled to one another. Although shown with a rotatable tongue 32c and groove 32d type connection, upper and lower housing portions 32a, 32b can be coupled together using any suitable securement technique such as fastening, snap-fit, friction-fit, adhesive, etc., for example. Housing 32 further support a seal assembly 32e having any number and/or type of seal and/or valve (e.g., disc seals, duckbill, etc.) for effectuating sealing receipt of any number and/or type of surgical instrumentation therethrough such as obturator 20, surgical stapler, forceps, clip applier, endoscope, etc., for example. Seal assembly 32e, or portions thereof, may be selectively removable and replaceable from housing 32 when upper housing portion 32a is removed from lower housing portion 32b.

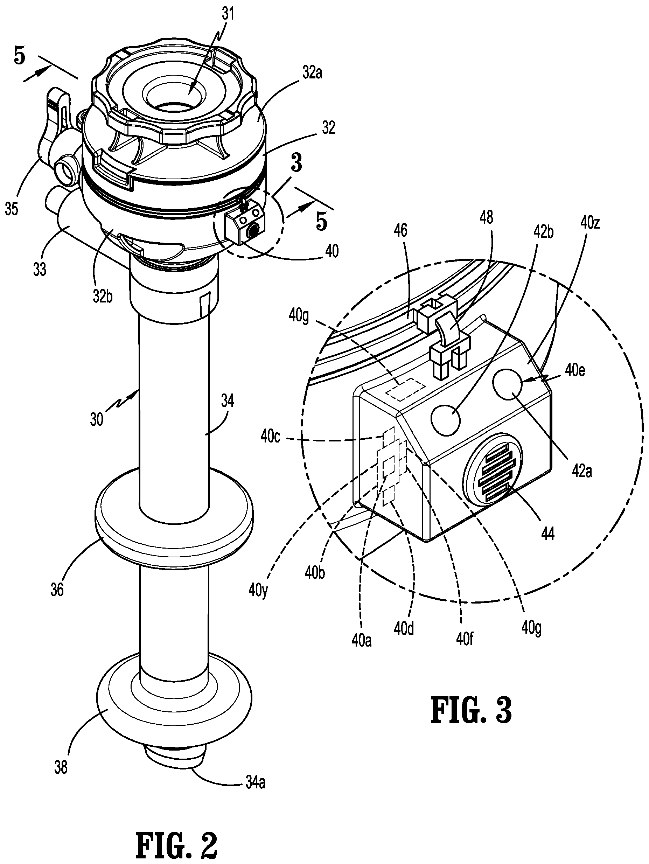

Cannula 34 of trocar assembly 30 extends to a distal tip 34a through which central passage 31 extends. Cannula 34 supports a stopper 36 and an expandable anchor 38 that disposed in spaced relation relative to one another along a length of cannula 34. Stopper 36 is configured to control an insertion depth of cannula 34. Stopper 36 may be movably positioned along cannula. In some aspects, stopper 36 is threadedly coupled to cannula 34 to adjust a longitudinal distance between stopper 36 and anchor 38 and/or housing 32 of trocar assembly 30. Expandable anchor 38 (e.g., a balloon) is movable between an unexpanded (e.g., uninflated) position (FIG. 1) to enable cannula 34 to advance through a natural or artificial opening "O" (e.g., incision), and an expanded (e.g., inflated) position (FIG. 2) to secure cannula 34 to tissue "T" when inserted through opening "O." Housing 32 supports an inflation port 33 in fluid communication with expandable anchor 38 to enable selective inflation and/or deflation of expandable anchor 38 with, for example, inflation fluid such as saline (not shown) from an inflation source (e.g., a syringe, not shown). Housing 32 also supports an insufflation assembly 35 for selectively enabling insufflation fluid (e.g., saline) to be passed through trocar 30 to selectively insufflate a body cavity beneath tissue "T." Housing 32 also supports a force sensor system 40 that is configured to alert a clinician when a force applied through the trocar system 10 exceeds a threshold force.

With reference to FIG. 3, force sensor system 40 includes a control unit 40z that supports a controller 40x and a power source 40y (e.g., battery or smart battery). Control unit 40z further includes one or more output devices, which may be in the form of a display 40e, and can include, for instance, LEDs 42a, 42b and speaker 44. In aspects, control unit 40z may include one or more input devices (e.g., graphical user interface such as touch screen, buttons, keyboard, etc.). Controller 40x includes memory 40a (and/or storage device) and a processor 40b. Memory 40a may store one or more applications 40f and/or data 40g. Application 40f (which may be a set of executable instructions) may, when executed by processor 40b, cause processor 40b to perform one or more operations associated with the instructions stored thereon. For instance, processor 40b may cause at least one of LED's 42a, 42b to light up. Application 40f may also provide the interface between one or more components of the system. Memory 12a may include any non-transitory computer-readable storage media for storing data and/or software (instructions) executable by processor 40b and which controls the operation of various components of the system, when in communication therewith.

In aspects, memory 40a may include one or more solid-state storage devices such as flash memory chips. Alternatively, and/or additionally, to the one or more solid-state storage devices, memory 40a may include one or more mass storage devices connected to processor 40b through a mass storage controller (not shown) and a communications bus (not shown). Although the description of computer-readable media contained herein refers to a solid-state storage, it should be appreciated by those skilled in the art that computer-readable storage media can be any available media that can be accessed by processor 40b. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by the processor 40b of this disclosure.

In aspects, force sensor system 40 may include a network interface 40c. Network interface 40c may be configured to connect to a network such as a local area network (LAN) including a wired network and/or a wireless network, a wide area network (WAN), a wireless mobile network, a Bluetooth network, and/or the Internet.

In aspects, force sensor system 40 may include an input/output module 40d. Input/output module 40d may include an input device through which a user may interact with a remote computing device of this disclosure, such as, for example, a mouse, keyboard, foot pedal, touch screen, and/or voice interface. Input/output module 40d may include an output device which may include any connectivity port or bus, such as, for example, parallel ports, serial ports, universal serial busses (USB), or any other similar connectivity port.

With continued reference to FIGS. 3-5, force sensor system 40 further includes a force sensor 46 (e.g., a piezoelectric sensor) and an electrical connector 48 (e.g., flexible ribbon cable) that couples force sensor 46 to control unit 40z. Force sensor 46 is disposed or sandwiched between upper and lower housing portions 32a, 32b of housing 32. Force sensor 46 may have an annular or ring configuration. Force sensor 46 further includes electrical contacts 46a, 46b that couple to a first end of electrical connector 48 and control unit 40z includes electrical contacts 49a, 49b that couple to a second end of electrical connector 48. Electrical contacts 49a, 49b of control unit 40z are disposed in electrical communication with controller 40x and a power source 40y of control unit 40z.

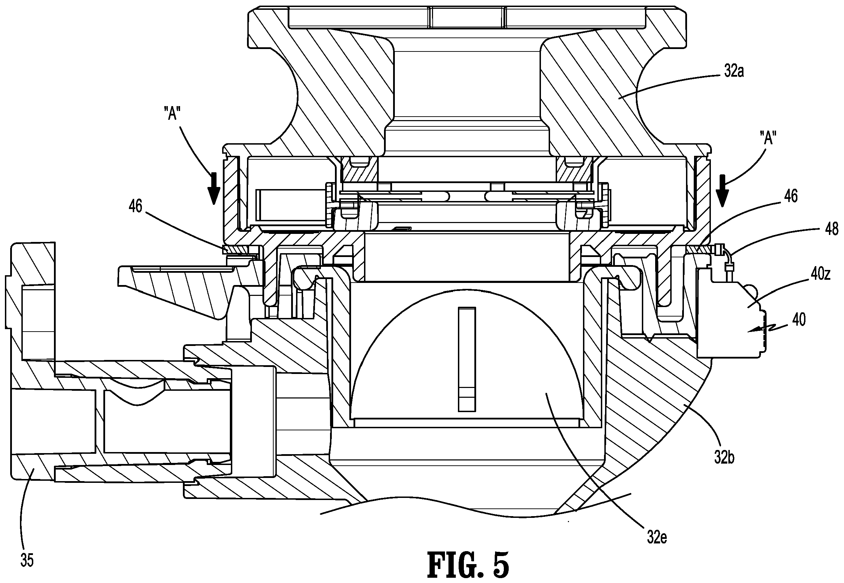

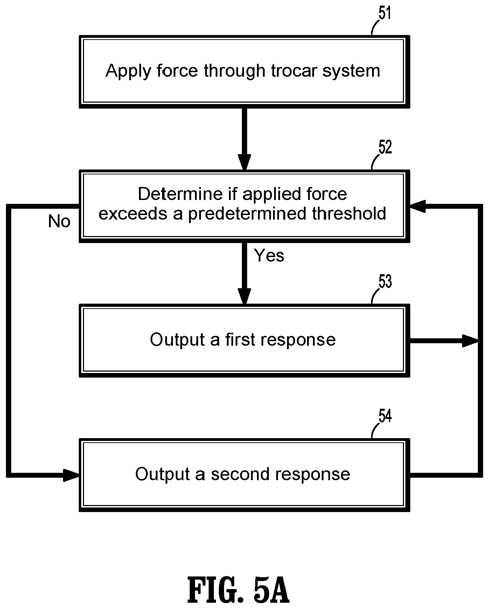

Referring to FIGS. 1-5A, in use, with trocar assembly 30 inserted in the tissue "T," one or more surgical instruments, such as obturator 20, can be introduced through the tissue "T" via trocar assembly 30 for accessing a surgical site within, for example, a body cavity below the tissue "T." In order to reach a portion of a surgical site that is farther than the length of obturator 20 or other surgical instrument, rather than moving the location of the trocar assembly 30, a clinician can apply a downward force/pressure through trocar assembly 30 toward the tissue "T" (e.g., against counterforce from the tissue "T") to attempt to move the trocar assembly 30 and/or surgical instrument (e.g., obturator 20) closer to a desired location within a body cavity, as indicated by arrows "A" shown in FIGS. 1 and 5, and indicated as step 51 in FIG. 5A.

With continued reference to FIG. 5A, in step, 52, force sensor system 40 senses an amount of applied pressure and communicates an electrical signal indicative of the amount of applied pressure to controller 40x. Controller 40x determines if the amount of applied pressure/force exceeds a predetermined threshold, by, for example, comparing the amount of applied pressure/force to the predetermined threshold, which may be stored on memory. If the amount of applied pressure/force exceeds the predetermined threshold, memory 40a can have instructions stored thereon, which when executed by processor 40b, cause control unit 40z to output a signal or first response through display 40ea that the applied pressure exceeds the predetermined threshold as indicated in step 53. For instance, LED 42a, which may be any suitable color, such as red, may be lit up to indicate that the applied pressure/force exceeds the predetermined threshold. In step 54, if the pressure/force does not exceed the predetermined threshold, LED 42b, which may be any suitable color, such as green, may be lit up to indicate that the applied pressure/force does not exceed the predetermined threshold so that the clinician knows that additional pressure/force may be added (second response). Additionally, and/or alternatively, memory 40a can have instructions stored thereon, which when executed by processor 40b, cause control unit 40z to output an audio message that is emitted from speaker 44 indicating, for example, that the amount of applied pressure/force is too much or is sufficient (e.g., safe).

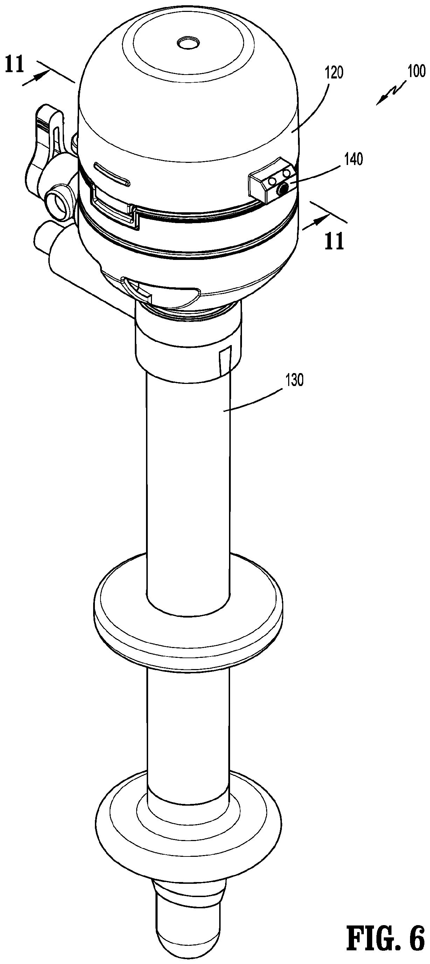

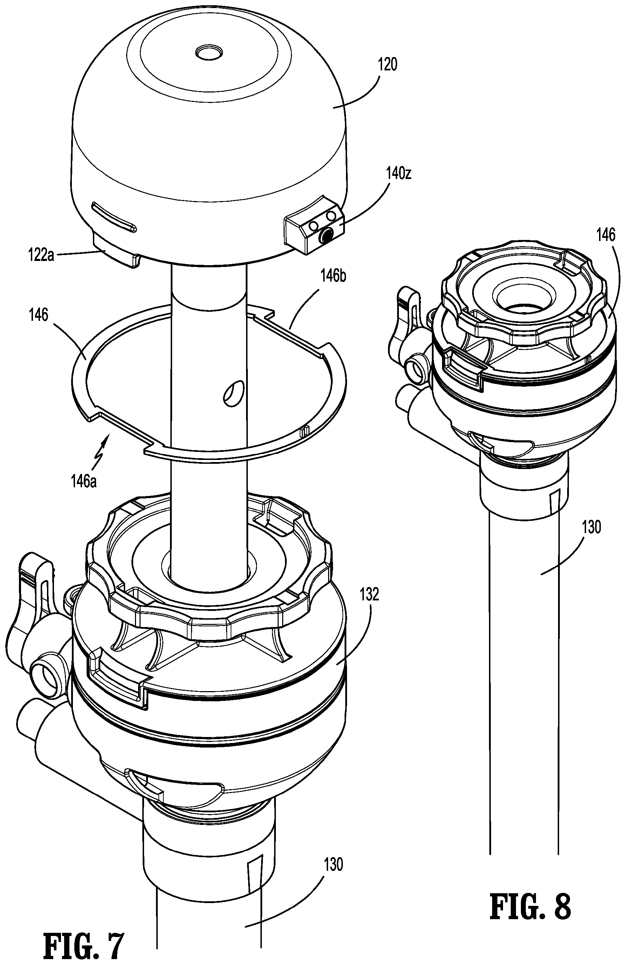

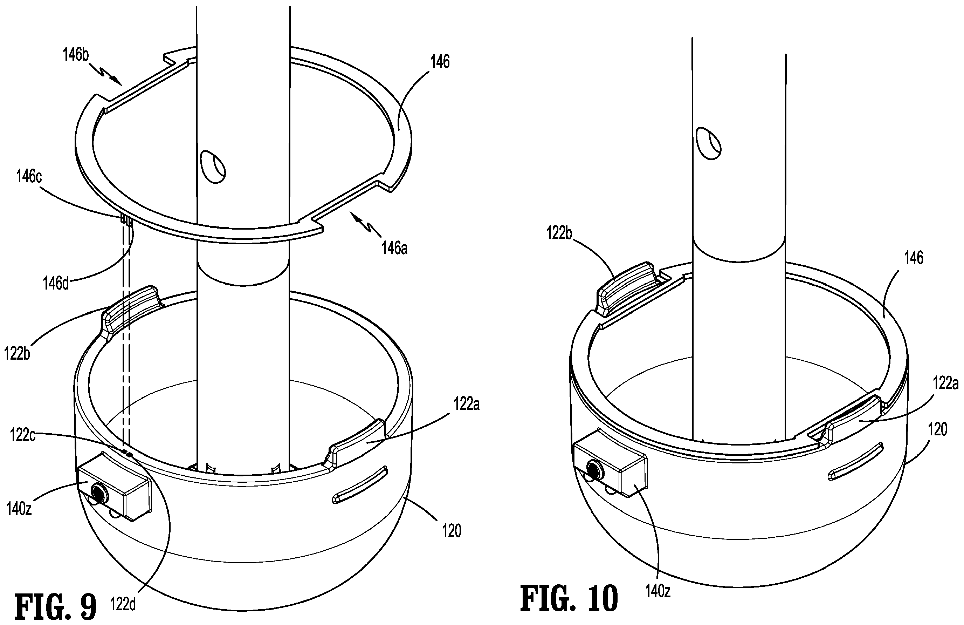

Turning now to FIGS. 6-11, another a trocar system, generally referred to as trocar system 100, is shown. Trocar system 100 is similar to trocar system 10. Trocar system 100 includes an obturator 120 and a trocar assembly 130 the receives obturator 120 therein. Obturator 120 is similar to obturator 20 and trocar assembly 130 is similar to trocar 30; however, trocar system 100 includes a force sensor system 140. Force sensor system 140 includes a control unit 140z secured to obturator 120 and a force sensor 146 that mounts to an upper housing portion 132 of trocar assembly 130. Control unit 140z is similar to control unit 40z and force sensor 146 is similar to force sensor 46. Force sensor 146 includes notches 146a, 146b for receiving tabs 122a, 122b of obturator 120. Force sensor 146, which may be a piezoelectric transducer, further includes electrical contacts 146c, 146d extending therefrom for electrically coupling to electrical contacts 122c, 122d of obturator 120. One or both electrical contacts 146c, 146d of force sensor 146 may be received within one or both electrical contacts 122c, 122d of obturator 120 (and/or vice versa) to physically couple force sensor 146 to obturator 120. Electrical contacts 122c, 122d are disposed in electrical communication with control unit 140z.

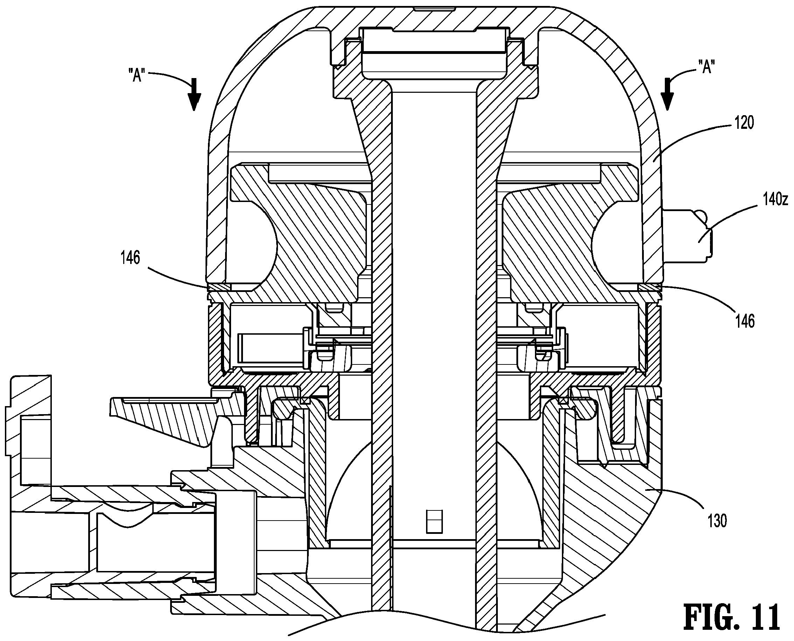

Referring to FIG. 11, in use, similar to that described above with regard to trocar system 10, force/pressure can be applied through trocar system 100, namely, through obturator 120, force sensor system 140, and/or trocar assembly 120 in an attempt to reach a desired location, as indicated by arrows "A." Then, as detailed, for example, similar to that that discussed above with respect to force sensor system 40, and illustrated in FIG. 5A, force sensor system 140 determines whether the amount of applied pressure/force is too much or is sufficient (e.g., safe).

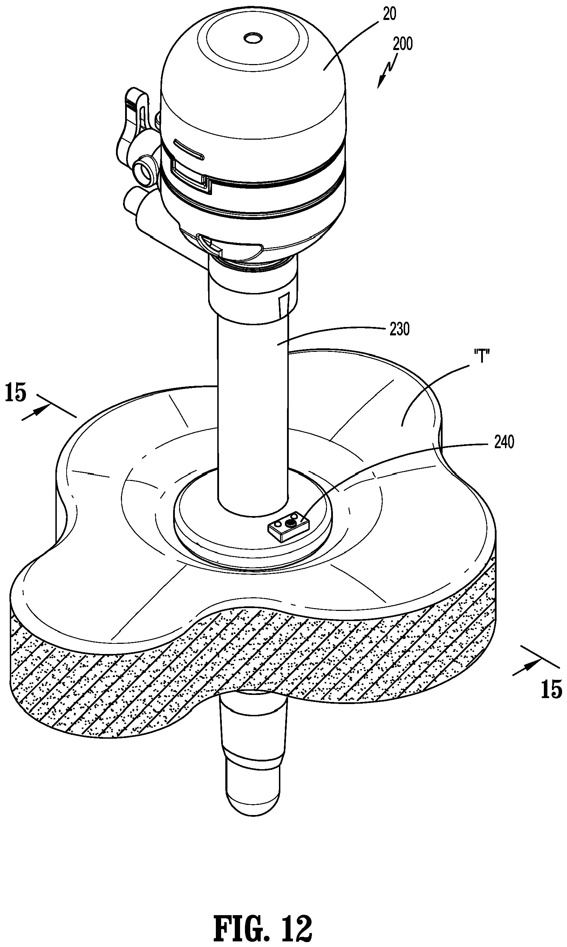

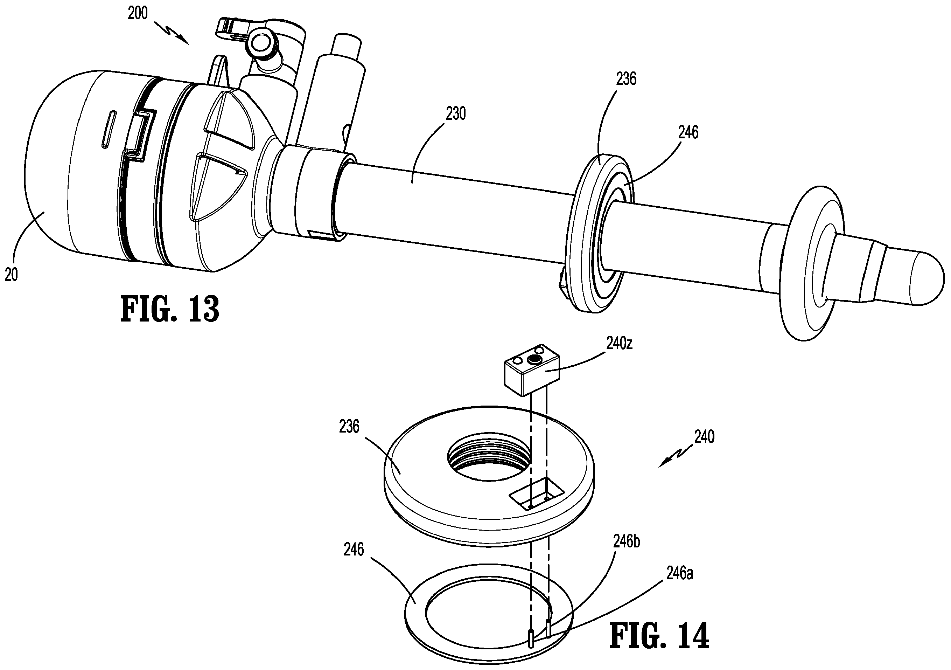

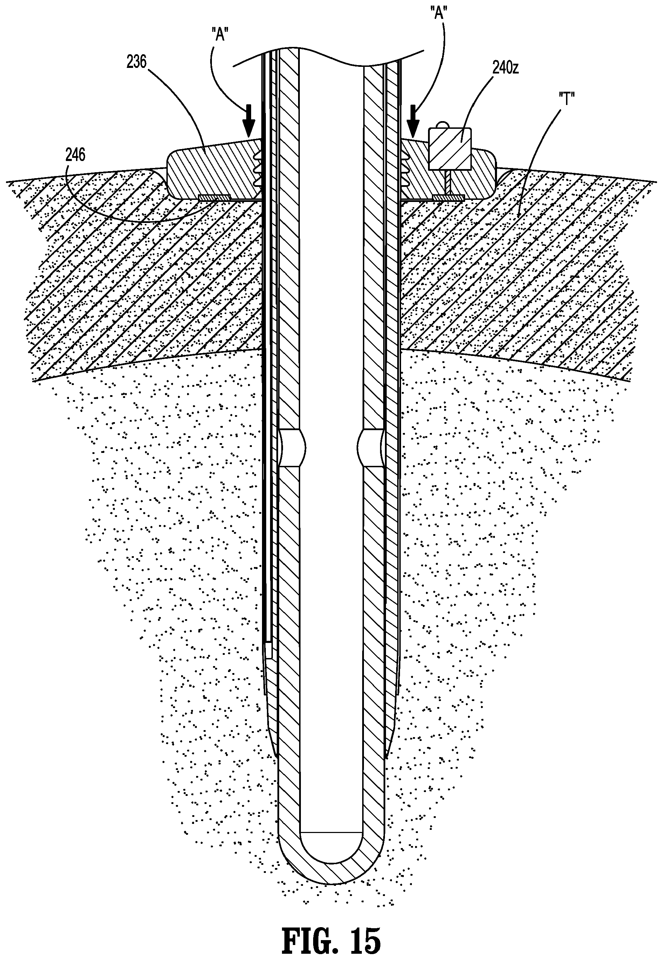

Turning now to FIGS. 12-15, still another trocar system, generally referred to as trocar system 200, is shown. Trocar system 200 is similar to trocar systems 10 and 100, and includes obturator 20 and trocar assembly 230 for receiving obturator 20. Trocar assembly 230 is similar to trocar assembly 30 and includes a stopper 236 supporting a force sensor system 240. Stopper 236 includes a control unit 240z, similar to control units 140z, 40z, and supports a force sensor 246 on a bottom surface of stopper 236. Force sensor 246 includes electrical contacts 246a, 246b that engage with corresponding electrical contacts of control unit 240z, and which may be secured to stopper 236.

In use, similar that that described above with regard to trocar systems 10, 100 force/pressure can be applied through force sensor system 240 of trocar system 200 in an attempt to reach a desired location, as indicated by arrows "A." Then, as detailed, for example, similar to that that discussed above with respect to force sensor system 40, and illustrated in FIG. 5A, force sensor system 240 determines whether the amount of applied pressure/force is too much or is sufficient (e.g., safe).

The various trocars disclosed herein may also be configured for use with robotic surgical systems, and what is commonly referred to as "Telesurgery." Such systems employ various robotic elements to assist the clinician and allow remote operation (or partial remote operation) of surgical instrumentation. Various robotic arms, gears, cams, pulleys, electric and mechanical motors, etc. may be employed for this purpose and may be designed with a robotic surgical system to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of clinicians may prep the patient for surgery and configure the robotic surgical system with one or more of the instruments disclosed herein while another clinician (or group of clinicians) remotely controls the instruments via the robotic surgical system. As can be appreciated, a highly skilled clinician may perform multiple operations in multiple locations without leaving his/her remote console which can be both economically advantageous and a benefit to the patient or a series of patients. For a detailed description of exemplary medical work stations and/or components thereof, reference may be made to U.S. Patent No. 8,828,023, and PCT Application Publication No. WO2016/025132, the entire contents of each of which are incorporated by reference herein.

For a more detailed description of similar trocars, one or more components of which can be included with, or modified for use with, the disclosed structure, reference can be made to U.S. Patent No. 5,807,338, filed October 20, 1995 and U.S. Patent No. 5,603,702, filed on August 8, 1994, the entire contents of each of which are incorporated by reference herein.

Moreover, the disclosed structure can include any suitable mechanical, electrical, and/or chemical components for operating the disclosed system or components thereof. For instance, such electrical components can include, for example, any suitable electrical and/or electromechanical, and/or electrochemical circuitry, which may include or be coupled to one or more printed circuit boards. As appreciated, the disclosed controller can include, for example, a "computing device," "processor," "digital processing device" and like terms, and which are used to indicate a microprocessor or central processing unit (CPU). The CPU is the electronic circuitry within a computer that carries out the instructions of a computer program by performing the basic arithmetic, logical, control and input/output (I/O) operations specified by the instructions, and by way of non-limiting examples, include server computers. In some aspects, the controller includes an operating system configured to perform executable instructions. The operating system is, for example, software, including programs and data, which manages hardware of the disclosed apparatus and provides services for execution of applications for use with the disclosed apparatus. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. In some aspects, the operating system is provided by cloud computing.

In some aspects, the term "controller" may be used to indicate a device that controls the transfer of data from a computer or computing device to a peripheral or separate device and vice versa, and/or a mechanical and/or electromechanical device (e.g., a lever, knob, etc.) that mechanically operates and/or actuates a peripheral or separate device.

In aspects, the controller includes a storage and/or memory device. The storage and/or memory device is one or more physical apparatus used to store data or programs on a temporary or permanent basis. In some aspects, the controller includes volatile memory and requires power to maintain stored information. In various aspects, the controller includes non-volatile memory and retains stored information when it is not powered. In some aspects, the non-volatile memory includes flash memory. In certain aspects, the non-volatile memory includes dynamic random-access memory (DRAM). In some aspects, the non-volatile memory includes ferroelectric random-access memory (FRAM). In various aspects, the non-volatile memory includes phase-change random access memory (PRAM). In certain aspects, the controller is a storage device including, by way of non-limiting examples, CD-ROMs, DVDs, flash memory devices, magnetic disk drives, magnetic tapes drives, optical disk drives, and cloud-computing-based storage. In various aspects, the storage and/or memory device is a combination of devices such as those disclosed herein.

In various embodiments, the memory can be random access memory, read-only memory, magnetic disk memory, solid state memory, optical disc memory, and/or another type of memory. In various embodiments, the memory can be separate from the controller and can communicate with the processor through communication buses of a circuit board and/or through communication cables such as serial ATA cables or other types of cables. The memory includes computer-readable instructions that are executable by the processor to operate the controller. In various embodiments, the controller may include a wireless network interface to communicate with other computers or a server. In aspects, a storage device may be used for storing data. In various aspects, the processor may be, for example, without limitation, a digital signal processor, a microprocessor, an ASIC, a graphics processing unit ("GPU"), field-programmable gate array ("FPGA"), or a central processing unit ("CPU").

Although illustrated as part of the disclosed structure, it is also contemplated that a controller may be remote from the disclosed structure (e.g., on a remote server), and accessible by the disclosed structure via a wired or wireless connection. In aspects, where the controller is remote, it is contemplated that the controller may be accessible by, and connected to, multiple structures and/or components of the disclosed system.

The term "application" may include a computer program designed to perform particular functions, tasks, or activities for the benefit of a user. Application may refer to, for example, software running locally or remotely, as a standalone program or in a web browser, or other software which would be understood by one skilled in the art to be an application. An application may run on the disclosed controllers or on a user device, including for example, on a mobile device, an IOT device, or a server system.

In some aspects, the controller includes a display to send visual information to a user. In various aspects, the display is a cathode ray tube (CRT). In various aspects, the display is a liquid crystal display (LCD). In certain aspects, the display is a thin film transistor liquid crystal display (TFT-LCD). In aspects, the display is an organic light emitting diode (OLED) display. In certain aspects, on OLED display is a passive-matrix OLED (PMOLED) or active-matrix OLED (AMOLED) display. In aspects, the display is a plasma display. In certain aspects, the display is a video projector. In various aspects, the display is interactive (e.g., having a touch screen or a sensor such as a camera, a 3D sensor, a LiDAR, a radar, etc.) that can detect user interactions/gestures/responses and the like. In some aspects, the display is a combination of devices such as those disclosed herein.

The controller may include or be coupled to a server and/or a network. As used herein, the term "server" includes "computer server," "central server," "main server," and like terms to indicate a computer or device on a network that manages the disclosed apparatus, components thereof, and/or resources thereof. As used herein, the term "network" can include any network technology including, for instance, a cellular data network, a wired network, a fiber optic network, a satellite network, and/or an IEEE 802.11a/b/g/n/ac wireless network, among others.

In various aspects, the controller can be coupled to a mesh network. As used herein, a "mesh network" is a network topology in which each node relays data for the network. All mesh nodes cooperate in the distribution of data in the network. It can be applied to both wired and wireless networks. Wireless mesh networks can be considered a type of "Wireless ad hoc" network. Thus, wireless mesh networks are closely related to Mobile ad hoc networks (MANETs). Although MANETs are not restricted to a specific mesh network topology, Wireless ad hoc networks or MANETs can take any form of network topology. Mesh networks can relay messages using either a flooding technique or a routing technique. With routing, the message is propagated along a path by hopping from node to node until it reaches its destination. To ensure that all its paths are available, the network must allow for continuous connections and must reconfigure itself around broken paths, using self-healing algorithms such as Shortest Path Bridging. Self-healing allows a routing-based network to operate when a node breaks down or when a connection becomes unreliable. As a result, the network is typically quite reliable, as there is often more than one path between a source and a destination in the network. This concept can also apply to wired networks and to software interaction. A mesh network whose nodes are all connected to each other is a fully connected network.

In some aspects, the controller may include one or more modules. As used herein, the term "module" and like terms are used to indicate a self-contained hardware component of the central server, which in turn includes software modules. In software, a module is a part of a program. Programs are composed of one or more independently developed modules that are not combined until the program is linked. A single module can contain one or several routines, or sections of programs that perform a particular task.

As used herein, the controller includes software modules for managing various aspects and functions of the disclosed system or components thereof.

The disclosed structure may also utilize one or more controllers to receive various information and transform the received information to generate an output. The controller may include any type of computing device, computational circuit, or any type of processor or processing circuit capable of executing a series of instructions that are stored in memory. The controller may include multiple processors and/or multicore central processing units (CPUs) and may include any type of processor, such as a microprocessor, digital signal processor, microcontroller, programmable logic device (PLD), field programmable gate array (FPGA), or the like. The controller may also include a memory to store data and/or instructions that, when executed by the one or more processors, cause the one or more processors to perform one or more methods and/or algorithms.

Any of the herein described methods, programs, algorithms, applications, or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

As can be appreciated, securement of any of the components of the disclosed systems can be effectuated using known securement techniques such welding, crimping, gluing, fastening, etc.

The phrases "in an aspect," "in aspects," "in various aspects," "in some aspects," or "in other aspects" may each refer to one or more of the same or different aspects in accordance with the present disclosure. Similarly, the phrases "in an embodiment," "in embodiments," "in various embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

Certain aspects of the present disclosure may include some, all, or none of the above advantages and/or one or more other advantages readily apparent to those skilled in the art from the drawings, descriptions, and claims included herein. Moreover, while specific advantages have been enumerated above, the various aspects of this disclosure may include all, some, or none of the enumerated advantages and/or other advantages not specifically enumerated above.

The aspects disclosed herein are examples of the disclosure and may be embodied in various forms. For instance, although certain aspects herein are described as separate aspects, each of the aspects herein may be combined with one or more of the other aspects herein. Specific structural and functional details disclosed herein are not to be interpreted as limiting, but as a basis for the claims and as a representative basis for teaching one skilled in the art to variously employ this disclosure in virtually any appropriately detailed structure. Like reference numerals may refer to similar or identical elements throughout the description of the figures.

Any of the herein described methods, programs, algorithms, or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

Persons skilled in the art will understand that the structures and methods specifically described herein and illustrated in the accompanying figures are non-limiting exemplary embodiments, and that the description, disclosure, and figures should be construed merely as exemplary of particular aspects. It is to be understood, therefore, that this disclosure is not limited to the precise aspects described, and that various other changes and modifications may be effected by one skilled in the art without departing from the scope or spirit of this disclosure. Additionally, it is envisioned that the elements and features illustrated or described in connection with one exemplary aspect may be combined with the elements and features of another without departing from the scope of this disclosure, and that such modifications and variations are also intended to be included within the scope of this disclosure. Indeed, any combination of any of the disclosed elements and features is within the scope of this disclosure. Accordingly, the subject matter of this disclosure is not to be limited by what has been particularly shown and described.

The invention may be described by reference to the following numbered paragraphs:-
1. A trocar system comprising:
   a trocar assembly including a housing and an elongated cannula extending from the housing;
   a force sensor system including:
      a force sensor supported on the trocar assembly;
      a control unit disposed in electrical communication with the force sensor;
      a processor; and
      a memory having instructions stored thereon, which when executed by the processor, cause the trocar system to:
         sense, with the force sensor, a force applied through the trocar system;
         determine, with the control unit, if the force exceeds a predetermined threshold; and
         through the control unit, selectively output a signal that indicates that the force exceeds the predetermined threshold.
2. The trocar system of paragraph 1, wherein the force sensor is a piezoelectric sensor.
3. The trocar system of paragraph 1, wherein the force sensor is supported on the housing of the trocar assembly.
4. The trocar system of paragraph 3, wherein the housing includes an upper housing portion and a lower housing portion, and wherein the force sensor is sandwiched between the upper and lower housing portions.
5. The trocar system of paragraph 3, wherein the force sensor is supported on an upper surface of the housing.
6. The trocar system of 1, wherein the elongated cannula supports a stopper that is configured to limit insertion depth of the trocar assembly into tissue.
7. The trocar system of paragraph 6, wherein the stopper supports the force sensor.
8. The trocar system of paragraph 6, wherein the control unit is supported on the stopper.
9. The trocar system of paragraph 1, wherein the control unit is supported on the housing.
10. The trocar system of paragraph 1, further comprising an obturator that is selectively received within trocar assembly.
11. The trocar system of paragraph 10, wherein the control unit is supported on the obturator.
12. A trocar system comprising:
   an obturator;
   a trocar assembly configured to receive the obturator therein;
   a force sensor system operatively coupled to at least one of the obturator or the trocar assembly, the force sensor system including:
      a force sensor ring;
      a control unit disposed in electrical communication with the force sensor ring;
      a processor; and
      a memory having instructions stored thereon, which when executed by the processor, cause the trocar system to:
         sense, with the force sensor ring, a force applied through the trocar system;
         determine, with the control unit, if the force exceeds a predetermined threshold; and
         through the control unit, selectively output a signal that indicates that the force exceeds the predetermined threshold.
13. The trocar system of paragraph 12, wherein the force sensor ring is a piezoelectric sensor.
14. The trocar system of paragraph 12, wherein the force sensor ring is supported on a housing of the trocar assembly.
15. The trocar system of paragraph 14, wherein the housing includes an upper housing portion and a lower housing portion, and wherein the force sensor ring is sandwiched between the upper and lower housing portions.
16. The trocar system of paragraph 14, wherein the force sensor ring is supported on an upper surface of the housing and positioned for engagement with the obturator.
17. The trocar system of 12, wherein trocar assembly includes an elongated cannula that supports a stopper configured to limit insertion depth of the trocar assembly into tissue.
18. The trocar system of paragraph 17, wherein the stopper supports the force sensor ring on a bottom surface thereof.
19. The trocar system of paragraph 18, wherein the control unit is supported on the stopper.
20. The trocar system of paragraph 12, wherein the control unit is supported on a housing of at least one of the obturator or the trocar assembly.

## Claims

1. A trocar system comprising:
a trocar assembly including a housing and an elongated cannula extending from the housing;
a force sensor system including:
a force sensor supported on the trocar assembly;
a control unit disposed in electrical communication with the force sensor;
a processor; and
a memory having instructions stored thereon, which when executed by the processor, cause the trocar system to:
sense, with the force sensor, a force applied through the trocar system;
determine, with the control unit, if the force exceeds a predetermined threshold; and
through the control unit, selectively output a signal that indicates that the force exceeds the predetermined threshold.

2. The trocar system of claim 1, wherein the force sensor is a piezoelectric sensor.

3. The trocar system of claim 1 or claim 2, wherein the force sensor is supported on the housing of the trocar assembly; preferably wherein the housing includes an upper housing portion and a lower housing portion, and wherein the force sensor is sandwiched between the upper and lower housing portions.

4. The trocar system of claim 3, wherein the force sensor is supported on an upper surface of the housing.

5. The trocar system of any preceding claim, wherein the elongated cannula supports a stopper that is configured to limit insertion depth of the trocar assembly into tissue; preferably wherein the stopper supports the force sensor.

6. The trocar system of claim 5, wherein the control unit is supported on the stopper.

7. The trocar system of any preceding claim, wherein the control unit is supported on the housing and/or further comprising an obturator that is selectively received within trocar assembly.

8. The trocar system of claim 7, wherein the control unit is supported on the obturator.

9. A trocar system comprising:
an obturator;
a trocar assembly configured to receive the obturator therein;
a force sensor system operatively coupled to at least one of the obturator or the trocar assembly, the force sensor system including:
a force sensor ring;
a control unit disposed in electrical communication with the force sensor ring;
a processor; and
a memory having instructions stored thereon, which when executed by the processor, cause the trocar system to:
sense, with the force sensor ring, a force applied through the trocar system;
determine, with the control unit, if the force exceeds a predetermined threshold; and
through the control unit, selectively output a signal that indicates that the force exceeds the predetermined threshold.

10. The trocar system of claim 9, wherein the force sensor ring is a piezoelectric sensor; preferably wherein the force sensor ring is supported on a housing of the trocar assembly.

11. The trocar system of claim 10, wherein the housing includes an upper housing portion and a lower housing portion, and wherein the force sensor ring is sandwiched between the upper and lower housing portions.

12. The trocar system of claim 10, wherein the force sensor ring is supported on an upper surface of the housing and positioned for engagement with the obturator.

13. The trocar system of any of claims 8 to 12, wherein trocar assembly includes an elongated cannula that supports a stopper configured to limit insertion depth of the trocar assembly into tissue.

14. The trocar system of claim 13, wherein the stopper supports the force sensor ring on a bottom surface thereof; and/or wherein the control unit is supported on the stopper.

15. The trocar system of claim 8, wherein the control unit is supported on a housing of at least one of the obturator or the trocar assembly.
